# EUROPÄISCHE PATENTSCHRIFT

(11) **EP 0 419 610 B1**
(45) Veröffentlichungstag und Bekanntmachung des Hinweises auf die Patenterteilung: **15.09.1993**
(21) Anmeldenummer: 90904594.0
(22) Anmeldetag: 16.03.1990
(51) Int. Cl.: C07C 69/732, C07C 67/343, A61K 31/215, C07C 69/007, C07C 59/48, C07C 33/30

(54) **NEUE LEUKOTRIEN-B4-DERIVATE, VERFAHREN ZU IHRER HERSTELLUNG UND IHRE VERWENDUNG ALS ARZNEIMITTEL**
NEW LEUKOTRIENE B4 DERIVATIVES, PROCESS FOR PRODUCING THEM AND THEIR USE AS DRUGS
NOUVEAUX DERIVES DE LEUCOTRIENE B4, PROCEDE POUR LA PREPARATION, ET LEUR UTILISATION COMME MEDICAMENTS

(30) Priorität: 17.03.1989 DE 3909327
(43) Veröffentlichungstag der Anmeldung: 03.04.1991
(73) Patentinhaber: SCHERING AKTIENGESELLSCHAFT, 59192 Bergkamen (DE)
(72) Erfinder: BUCHMANN, Bernd, D-1000 Berlin 21 (DE); SKUBALLA, Werner, D-1000 Berlin 28 (DE); HEINDL, Joseph, D-1000 Berlin 38 (DE); FRÖHLICH, Wolfgang, D-1000 Berlin 31 (DE); EKERDT, Roland, D-1000 Berlin 28 (DE)
(86) Internationale Anmeldenummer: DE9000211
(87) Internationale Veröffentlichungsnummer: WO9011271

(56) Entgegenhaltungen:
- EP-A- 0 109 225
- EP-A- 0 296 580

## Beschreibung

Die Erfindung betrifft neue Leukotrien-B₄-Derivate, Verfahren zu ihrer Herstellung sowie ihre Verwendung als Arzneimittel.

Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird.

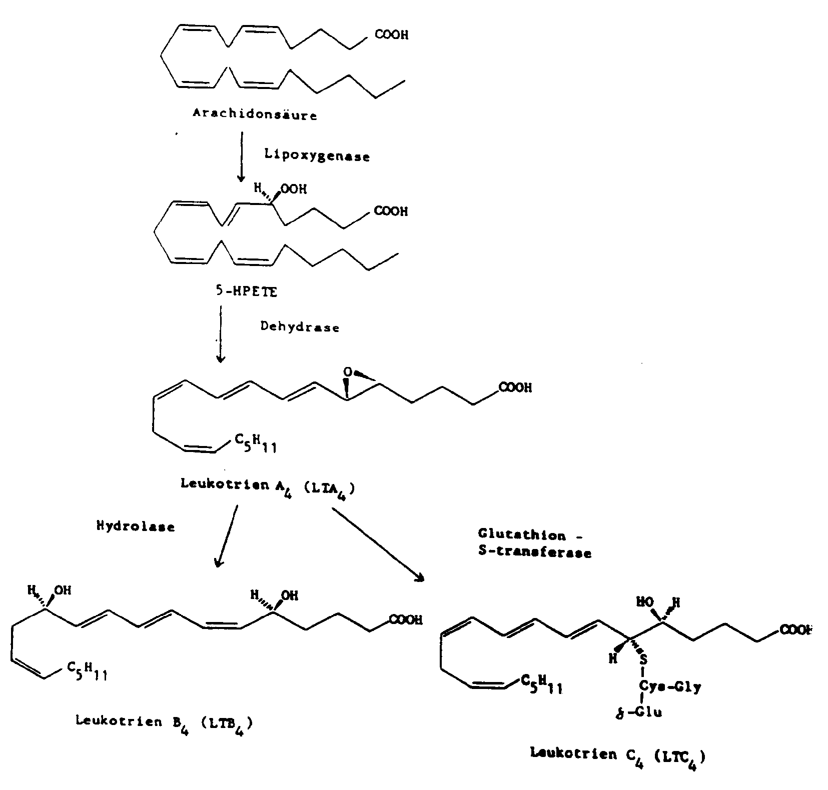

Die Nomenklatur der Leukotriene kann folgenden Arbeiten entnommen werden: a) B. Samuelsson et al., Prostaglandins 19, 645 (1980); 17, 785 (1979). b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic Press 1984. b) J.W. Gillard et al., Drugs of the Future 12, 453 (1987). c) B. Samuelsson et al., Science 237, 1171 (1987). d) C.W. Parker, Drug Development Research 10, 277 (1987). Hieraus ergibt sich, daß LTB₄ ein wichtiger Entzündungsmediator für entzündliche Erkrankungen ist, bei denen Leukozyten in das erkrankte Gewebe einwandern.

Vom LTB₄ weiß man, daß es die Adhäsion von Leukozyten an die Blutgefäßwand verursacht. LTB₄ ist chemotaktisch wirksam, d.h. es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wurde. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrienkonzentration sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentration wurden beispielsweise in der Haut von Patienten mit Psoriasis oder atopischer Dermatitis gemessen.

Weiterhin sind Leukotriene und LTB₄ insbesondere bei Arthritis chronischer Lungenerkrankung (z.B. Asthma), Rhinitis und entzündlichen Darmerkrankungen beteiligt.

Antagonisten gegen LTB₄ selbst oder Inhibitoren jener Enzyme, die an der Synthese des LTB₄ beteiligt sind, können als spezifische Heilmittel, besonders gegen Krankheiten, die mit Entzündungen und allergischen Reaktionen einhergehen, wirksam sein.

Neben therapeutischen Möglichkeiten, die sich aus einer Antagonisierung des LTB₄ mit LTB₄-Analoga ableiten lassen, konnte kürzlich auch die Nützlichkeit und potentielle Anwendung von Leukotrien B₄-Agonisten zur Behandlung von Pilzerkrankungen der Haut gezeigt werden (H. Kayama, Prostaglandins 34, 797 (1988)).

Der Ersatz der chemisch und metabolisch labilen cis-4⁶^{,7}-Doppelbindung des LTB₄ durch einen 1,2-substituierten Phenylring führt zu den stabileren 6,7-Interphenylen-Leukotrienen, wobei je nach Strukturveränderung der funktionellen Gruppen und je nach Gewebeart Antagonisten, Agonisten und Partialantagonisten erhalten werden. Es wurde nun gefunden, daß durch die Substitution der 5-Hydroxygruppe durch ein Wasserstoffatom und durch weitere Derivatisierung der funktionellen Gruppen LTB₄-Analoga erhalten werden, die die Wirkung des natürlichen LTB₄ stark antagonisieren. Wirkdauer und Selektivität der neuen Verbindungen konnten durch geringere Oxidationsempfindlichkeit oder fehlende Laktonisierungstendenz aufgrund der nicht vorhandenen 5-Hydroxygruppe weiter verbessert werden.

Die Erfindung betrifft neue Leukotrien-B₄-Analoge der Formel I, 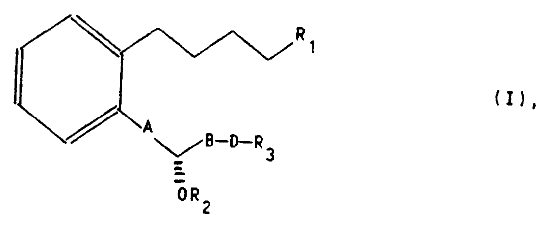
worin,
- R₁: den Rest CH₂OH, CH₃, CF₃, oder COOR₄ mit R₄ in der Bedeutung eines Wasserstoffatoms, eines Alkylrestes mit 1-10 C-Atomen, eines Cycloalkylrestes mit 3-10 C-Atomen, eines gegebenenfalls durch 1-2 Chlor, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrestes mit 6-10 C-Atomen, eines -CH₂-CO-Aryl-Restes mit 6-10 C-Atomen für Aryl oder eines 5-6-gliedrigen heterocyclischen Restes mit wenigstens 1 Heteroatom, den Rest CONHR₅ mit R₅ in der Bedeutung des Restes R₄ oder eines Alkanoyl- oder Alkansulfonylrestes mit 1-10 C-Atomen,
- A: eine trans,trans-CH=CH-CH=CH-, trans-CH₂-CH₂-CH=CH- oder Tetramethylengruppe,
- B: eine geradkettige oder verzweigtkettige gesättigte oder ungesättigte Alkylengruppe mit bis zu 10 C-Atomen, die gegebenenfalls durch Fluor substituiert sein kann, oder die Gruppe 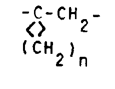 mit n = 1, 2 oder 3,
- D: eine Direktverbindung, Sauerstoff, Schwefel, eine -C≡C-Gruppe oder eine -CH=CR₆-Gruppe mit R₆ als Wasserstoff, C₁-C₅-Alkyl, Chlor oder Brom,
- B und D: gemeinsam eine direkte Bindung,
- R₂: ein Wasserstoffatom oder einen Säurerest einer organischen Säure mit 1-15 C-Atomen und
- R₃: ein Wasserstoffatom, einen Alkylrest mit 1-10 C-Atomen, einen durch Chlor oder Brom substituierten Alkylrest mit 1-10 C-Atomen, einen Cycloalkylrest mit 3-10 C-Atomen, einen gegebenenfalls durch 1-2 Chlor-, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrest mit 6-10 C-Atomen oder einen 5-6-gliedrigen heterocyclischen Rest mit wenigstens 1 Heteroatom bedeuten und, falls R₄ die Bedeutung eines Wasserstoffatom hat, deren Salze mit physiologisch verträglichen Basen und deren Cyclodextrinclathrate.

Als Alkylgruppen R₄ kommen gerad- oder verzweigtkettige Alkylgruppen mit 1-10 C-Atomen in Betracht, wie beispielsweise Methyl, Ethyl, Propyl, Butyl, Isobutyl, tert.-Butyl, Pentyl, Neopentyl, Hexyl, Heptyl, Decyl. Die Alkylgruppen R₄ können gegebenenfalls ein- bis mehrfach substituiert sein durch Halogenatome, Alkoxygruppen, gegebenenfalls substituierte Aryl- bzw. Aroylgruppen, Dialkyl amino und Trialkylammonium, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R₄ sind solche mit 1-4 C-Atomen zu nennen.

Als Arylgruppen R₄ kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen, eine Chlormethyl-, Fluormethyl-, Trifluormethyl,- Carboxyl-, Hydroxy- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hydroxy oder Alkoxygruppen.

Die Cycloalkylgruppe R₄ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl.

Als heterocyclische Gruppen R₄ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest R₅ kommen physiologisch verträgliche Säurereste in Frage. Bevorzugte Säuren sind organische Carbonsäuren und Sulfonsäuren mit 1-10 Kohlenstoffatomen, die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für die Substituenten seinen Alkyl-, Hydroxy-, Alkoxy-, Oxo- oder Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Trimethylessigsäure, Diethylessigsäure, tert.-Butylessigsäure, Cyclopropylessigsäure, Cyclopentylessigsäusäure, Cyclohexylessigsäure, Cyclopropancarbonsäure, Cyclohexancarbonsäure, Phenylessigsäure, Phenoxyesigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy,- Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste und Alkansulfonylreste werden solche mit bis zu 6 Kohlenstoffatomen betrachtet. Als Sulfonsäuren kommen beispielsweise Methansulfonsäure, Ethansulfonsäure, Isopropansulfonsäure, β-Chlorethansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diethylaminosulfonsäure, N,N-Bis-(β-chlorethyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Als Alkylgruppen R₃ kommen gerad- und verzweigtkettige, gesättigte und ungesättigte Alkylreste, vorzugsweise gesättigte, mit 1-10, insbesondere 1-6 C-Atomen, in Frage, die gegebenenfalls durch gegebenenfalls substituiertes Aryl substituiert sein können. Beispielsweise genannt seien Methyl-, Ethyl-, Propyl-, Butyl-, Isobutyl-, tert.,-Butyl-, Pentyl-, Hexyl-, Heptyl-, Octyl-, Butenyl-, Isobutenyl-, Propenyl-, Pentenyl-, Benzyl-, m- und p-Chlorbenzylgruppen. Sind die Alkylgruppen R₃ Halogen-substituiert, kommen als Halogene Fluor, Chlor und Brom in Frage.

Die Cycloalkylgruppe R₃ kann im Ring 3-10, vorzugsweise 3-6 Kohlenstoffatome enthalten. Die Ringe können durch Alkylgruppen mit 1-4 Kohlenstoffatomen substituiert sein. Beispielsweise genannt seinen Cyclopropyl, Cyclobutyl, Cyclopentyl, Cyclohexyl, Methyl-cyclohexyl.

Als substituierte bzw. unsubstituierte Arylgruppen R₃ kommen beispielsweise in Betracht: Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen eine Chlormethyl-, Fluormethyl-, Trifluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy oder Alkoxygruppen.

Als heterocyclische Gruppen R₃ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, u.a.

Als Alkylengruppe B kommen geradkettige oder verzweigtkettige, gesättigte und ungesättigte Alkylenreste mit bis zu 10 C-Atomen, vorzugsweise gesättigte mit 1-10, insbesondere mit 1-5 C-Atomen, in Frage, die gegebenenfalls durch Fluoratome substituiert sein können. Beispielsweise seien genannt; Methylen, Fluormethylen, Difluormethylen, Ethylen, 1,2-Propylen, Ethylethylen, Trimethylen, Tetramethylen, Pentamethylen, 1.1-Difluorethylen, 1-Fluorethylen, 1-Methyltetramethylen, 1-Methyl-tri-methylen, 1-Methylen-ethylen, 1-Methylen-tetramethylen.

Als Säurereste R₂ kommen physiologisch verträgliche Säurereste in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch und/oder in üblicher Weise substituiert sein. Als Beispiele für die Substituenten seien Alkyl-, Hydroxy-, Alkoxy, Oxo- oder Aminogruppen oder Halogenatome erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopentylessigsäure, Cyclohexylessigsäure, Cyclohexancarbonsäure, Phenylessigsäure, Phenoxyesigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy,- Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Säurereste R₂ kommen Acylreste mit bis zu 10 Kohlenstoffatomen in Betracht.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Die Leukotrien-B₄-Derivate der Formel I bilden mit α-, β-, γ-Cyclodextrin die bereits genannten Cyclodextrinclathrate.

Die Erfindung beinhaltet außerdem ein Verfahren zur Herstellung der Leukotrien-B₄-Derivate der Formel I, das dadurch gekennzeichnet ist, daß man einen Aldehyd der Formel II, 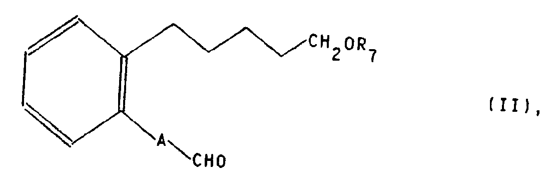
worin A die oben angegebene Bedeutung hat und R₇ einen leicht abspaltbaren Etherrest bedeutet, wie beispielsweise Dimethyl-tert.-butylsilyl, Trimethylsilyl-, Tribenzylsilyl-, Diphenyl-tert.-butylsilyl-, Tetrahydropyranyl-, Tetrahydrofuranyl- und α-Ethoxyethyl, um nur einige zu nennen, mit einer magnesiumorganischen Verbindung der Formel III,

X - Mg - B - D -R₃ (III),

worin X Chlor, Brom oder Jod und B, D und R₃ die oben angegebene Bedeutungen haben, umgesetzt und gegebenenfalls anschließend in beliebiger Reihenfolge Enantiomere getrennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R₁=COOR₅) verseift und/oder reduziert und/oder eine Carboxylgruppe (R₅=H) verestert und/oder eine freie Carboxylgruppe (R₅=H) in ein Amid (R₁=CONHR₆) überführt oder eine Carboxygruppe mit einer physiologisch verträglichen Base in ein Salz überführt.

Die Umsetzung der Verbindung der Formel II mit einer metallorganischen Verbindung der Formel III erfolgt in an sich bekannter Weise in einem inerten Lösungsmittel oder Lösungsmittelgemisch, wie zum Beispiel Diethylether, Tetrahydrofuran, Dioxan, Toluol, Dimethoxyethan, vorzugsweise Diethylether oder Tetrahydrofuran. Die Reaktion wird bei Temperaturen zwischen -100 °C und 60 °C, vorzugsweise bei -78 °C bis 0 °C durchgeführt.

Die Herstellung der für diese Umsetzung benötigten Verbindung der Formel III erfolgt durch Reaktion des entsprechenden Halogenids durch Umsetzung mit Magnesium.

Die Reduktion zu den Verbindungen der Formel I mit R₁ in der Bedeutung einer -CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30 °C bis zur Siedetemperatur des verwendeteten Lösungsmittels, vorzugsweise 0 °C bis 30 °C vorgenommen.

Die Veresterung der Alkohole der Formel I (R₂ = H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise Na-Hydrid, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid, DMSO bei Temperaturen über oder unter Raumtemperatur, zum Beispiel zwischen -80 °C bis 100 °C, vorzugsweise bei Raumtemperatur, vorgenommen werden.

Die Oxidation der 1-Hydroxygruppe wird nach den dem Fachmann bekannter Methoden vorgenommen. Als Oxidationsmittel können beispielsweise dienen; Pyridiniumdichromat (Tetrahedron Letters, 1979, 399), Jones-Reagenz (J. Chem. Soc. 1953, 2555) oder Platin/Sauerstoff (Adv. in Carbohydrate Chem. 17, 169 (1962) oder Collins-Oxidation und anschließende Jones-Oxidation. Die Oxidation mit Pyridiniumchromat wird bei Temperaturen von 0 °C bis 100 °C, vorzugsweise 20 °C bis 40 °C in einem gegen das Oxidationsmittel inerten Lösungsmittel, beispielsweise Dimethylformamid, durchgeführt.

Die Oxidation mit Jones-Reagenz wird bei Temperaturen von -40 °C bis +40 °C, vorzugsweise 0 °C bis 30 °C in Aceton als Lösungsmittel ausgeführt.

Die Oxidation mit Platin/Sauerstoff wird bei Temperaturen von 0 °C bis 60 °C, vorzugsweise 20 °C bis 40 °C in einem gegen das Oxidationsmittel inerten Lösungsmittel, wie z. B. Essigester, durchgeführt.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden.

Die Freisetzung der funktionell abgewandelten Hydroxygruppen erfolgt nach bekannten Methoden. Beispielweise wird die Abspaltung von Hydroxyschutzgruppen, wie beispielsweise des Tetrahydropyranylrestes, in einer wässrigen Lösung einer organischen Säure, wie z.B. Oxalsäure, Essigsäure, Propionsäure u.a., oder in einer wässrigen Lösung einer anorganischen Säure, wie z. B. Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind z. B. Alkohole, wie Methanol und Ethanol, und Ether, wie Dimethoxyethan, Dioxan und Tetrahydrofuran. Tetrahydrofuran wird bevorzugt angewendet. Die Abspaltung wird vorzugsweise bei Temperaturen zwischen 20 °C und 80 °C durchgeführt. Die Abspaltung der Silyletherschutzgruppen erfolgt beispielsweise mit Tetrabutylammoniumfluorid oder mit Kaliumfluorid in Gegenwart eines Kronenethers. Als Lösungsmittel sind beispielsweise geeignet Tetrahydrofuran, Diethylether, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohol kommen aliphatische Alkohole in Betracht, wie z.B. Methanol, Ethanol, Butanol usw., vorzugsweise Methanol. Als Alkalicarbonate und -hydroxyde seien Kalium- und Natriumsalze genannt. Bevorzugt sind die Kaliumsalze.

Als Erdkalicarbonate und -hydroxide sind beispielsweise geeignet Calciumcarbonat, Calciumhydroxyd und Bariumcarbonat. Die Umsetzung erfolgt bei -10 °C bis +70 °C, vorzugsweise bei +25 °C.

Die Einführung der Estergruppe 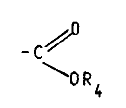
für R₁, bei welcher R₄ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffes in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389 - 394 (1954)].

Die Einführung der Estergruppe 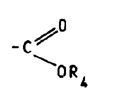
für R₁, bei welcher R₄ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30 °C und +50 °C, vorzugsweise bei 10 °C, durchgeführt.

Sollen im Primärprodukt enthaltene C=C-Doppelbindungen reduziert werden, erfolgt die Hydrierung nach an sich bekannten Methoden.

Die Hydrierung des Δ^{8,10}-Diensystems wird in an sich bekannter Weise bei tiefen Temperaturen, vorzugsweise bei etwa -20 °C bis +30 °C in einer Wasserstoffatmosphäre in Gegenwart eines Edelmetallkatalysators durchgeführt. Als Katalysator ist zum Beispiel 10 % Palladium auf Kohle geeignet.

Die Leukotrien-B₄-Derivate der Formel I mit R₄ in der Bedeutung eines Wasserstoffatoms können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes wird die LTB₄-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe 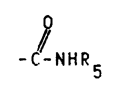
für R₁ erfolgt nach den dem Fachmann bekannten Methoden. Die Carbonsäuren der Formel I (R₄=H), werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amids oder mit Ammoniak (R₅=H) erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30 °C und +60 °C, vorzugsweise bei 0 °C bis 30 °C.

Eine weitere Möglichkeit für die Einführung der Amidgruppe 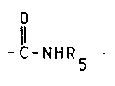
für R₁ besteht in der Umsetzung einer 1-Carbonsäure der Formel I (R₄=H), in der freie Hydroxygruppen gegebenenfalls intermediär geschützt sind, mit Verbindungen der Formel IV,

O = C = N - R₅ (IV),

worin R₅ die oben angegebene Bedeutung hat.

Die Umsetztung der Verbindung der Formel I (R₄=H) mit einem Isocyanat der Formel IV erfolgt gegebenenfalls unter Zusatz eines tertiären Amins, wie z.B. Triethylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80 °C bis 100 °C, vorzugsweise bei 0 °C bis 30 °C, vorgenommen werden.

Enthält das Ausgangsprodukt OH-Gruppen im Leukotrien-B₄-Rest so werden diese OH-Gruppen auch zur Reaktion gebracht. Werden letztlich Endprodukte gewünscht, die freie Hydroxylgruppen enthalten, geht man zweckmässigerweise von Ausgangsprodukten aus, in denen diese durch vorzugsweise leicht abspaltbare Ether- oder Acylreste intermediär geschützt sind.

Die als Ausgangsmaterial dienenden Verbindungen der Formel II können beispielsweise wie folgt hergestellt werden.

Die Hydroxygruppe des 4-Chlorbutanols wird in an sich bekannter Weise durch Reaktion mit einem entsprechend substituiertes Silylchlorid unter Basenkatalyse oder z.B. durch Reaktion mit einem entsprechend subsitutuierten Enolether unter Säurekatalyse in die Ether der Formel V überführt, wobei R₇ die oben angegebene Bedeutung besitzt

Cl - CH₂ - CH₂ - CH₂ - CH₂ - OR₇ (V).

Durch Alkylierung des dilithierten 2-Methylbenzylalkohols mit dem Chlorid der Formel V erhält man den Alkohol der Formel VI 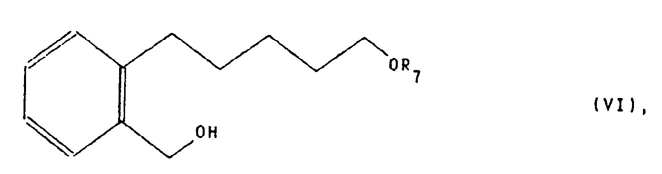
der durch Oxidation z.B. mit Braunstein in den Aldehyd der Formel VII überführt wird

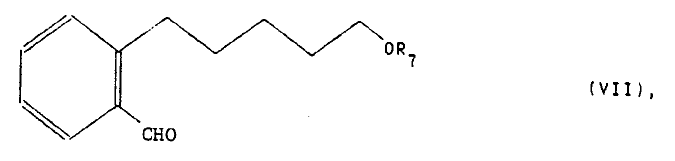

Durch eine Wittig-Horner-Olefinierung des Aldehyds der Formel VII mit dem Phosphonat der Formel VIII, anschließender Reduktion des Esters, beispielsweise mit DIBAH, Hydrierung der Doppelbindung, Oxidation des Alkohols mit z.B. Collinsreagenz zum Aldehyd und nochmalige Wittig-Horner Reaktion mit dem Phosphonat der Formel VIII, oder durch Wittig-Horner-Olefinierung des Aldehyds der Formel VII mit dem Phosphonat der Formel IX und einer Base und gegebenenfalls anschließender Hydrierung erhält man aus den Aldehyd der Formel VII die Ester 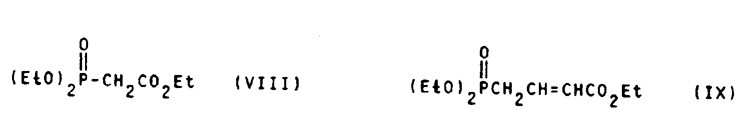
der Formel X, wobei A die oben angegebene Bedeutung besitzt.

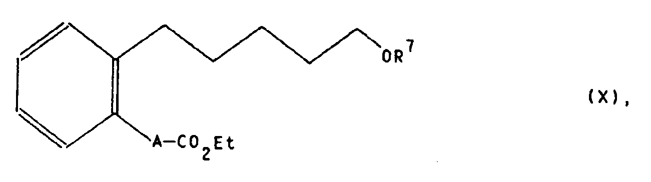

Als Basen für die vorstehende Wittig-Horner Olefinierung kommen beispielsweise K-tert.butylat, Diazabicyclononan bzw. -undecan oder Natriumhydrid in Frage.

Die Reduktion der Estergruppe, beispielsweise mit DIBAH und anschließende Oxidation des erhaltenen primären Alkohols z.B. mit Braunstein oder Collinsreagenz führt zum Aldehyd der Formel II.

Die Verbindungen der Formel I wirken antientzündlich und antiallergisch. Daneben besitzen sie antimykotische Eigenschaften. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien B₄-Derivate der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erythrodermie, Verbrennungen, Tinea, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigeneten Zusätzen in die gewünschte Applikationsform, wie zum Beispiel: Lösungen, Lotionen, Salben, Cremes oder Pflaster, überführt, In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise o,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Die neuen Leukotrien-B₄-Derivate können auch in Kombination, wie z.B mit Lipoxygenasehemmern, Cyclooxygenasehemmern, Prostacyclinagonisten, Thromhoxanantagonisten, Leukotrien D₄-antagonisten, Leukotrien E₄-antagonisten, Leukotrien F₄-antagonisten, Phosphodiesterasehemmern oder PAF-Antagonisten verwendet werden.

### Biespiel 1

5-[2-[(1E,3E)-(5RS)-5-Acetoxy-1,3-tridecadienyl]-phenyl]-pentan-1-ol

Zu 559 mg Magnesium tropft man bei 25 °C unter Argon eine Lösung aus 3,97 ml 1-Bromoctan in 7 ml Ether und rührt anschließend 30 min. bei 25 °C.

Zu einer Lösung von 301 mg (2E,4E)-5-[2-(5-Diphenyl-tert.-butylsilyloxypentyl)-phenyl]-2,4-pentadienal in 10 ml Ether werden bei -20 °C unter Argon 2 ml der oben erhaltenen Grignard-Lösung zugegeben und 40 min. bei -20 °C gerührt. Man gießt die Reaktionsmischung auf 50 ml einer gesättigten Ammoniumchloridlösung, extrahiert dreimal mit Ether, schüttelt die organische Phase mit Sole, trocknet über Natriumsulfat und dampft im Vakuum ein. Den so erhaltenen Rückstand chromatographiert man an Kieselgel. Mit Hexan/Methyl-tert.-butylether (9+1) erhält man 329 mg 5-[2-[(1E,3E)-(5RS)-5-Hydroxy-1,3-tridecadienyl]-phenyl]-pentan-1-ol-diphenyl-tert.-butylsilylether als farbloses Öl.

IR (CHCl₃): 3603, 3440 (breit), 2930, 990 cm⁻¹.

Zur Acetylierung fügt man zu einer Lösung von 310 mg des vorstehend beschriebenen Alkohols in 2 ml Pyridin 0,5 ml Essigäsureanhydrid und rührt 16 Stunden bei 25 °C. Anschließend engt man unter Zusatz von Toluol im Vakuum ein und chromatographiert den Rückstand an Kieselgel. Mit Hexan/1-5 % Methyl-tert.-butylether erhält man 265 mg 5-[2-[(1E,3E)-(5RS)-5-Acetoxy-1,3-tridecadienyl]-phenyl]-pentan-1-ol-diphenyl-tert.-butylsilylether als farbloses Öl.

IR (CHCl₃): 2932, 1728, 1245, 990 cm⁻¹.

Zur Silyletherspaltung gibt man zu einer Lösung von 200 mg des zuvor hergestellten Acetats in 4 ml Tetrahydrofuran 984 mg Tetrabutylammoniumfluoridtrihydrat und rührt 1,5 Stunden bwi 24 °C. Anschließend wird mit Ether verdünnt, mit Wasser gewaschen, über Natriumsulfat getrocknet und im Vakuum eingeengt. Der so erhaltene Rückstand wird über Kieselgel mit Hexan/Essigester (1+1) filtriert und die Lösung im Vakuum eingeengt. Anschließend wird das Rohprodukt an Kieselgel mit Hexan/10 - 50 % Essigester chromatographiert. Man erhält 92 mg der Titelverbindung als farbloses Öl.

IR (CHCl₃): 3625, 3480 (breit), 2930, 1728, 1248, 990 cm⁻¹.

Das Ausgangsmaterial für die obige Titelverbindung wird wie folgt hergestellt:
a) 4-Chlorbutan-1-ol-diphenyl-tert.-butylsilylether
   Zu einer Lösung von 60 g 4-Chlorbutan-1-ol und 94 g Imidazol in 700 ml Dimethylformamid gibt man bei 0 °C unter Argon 168 g Diphenyl-tert.-butylsilylchlorid zu und rührt dann 16 Stunden bei 24 °C. Anschließend verdünnt man die Reaktionsmischung mit Hexan/Ether (8+2) und wäscht die organische Phase nacheinander einmal mit Wasser, zweimal mit 10 %iger Schwefelsäure und einmal mit halbkonzentrierter Natriumchloridlösung. Nach dem Trocknen über Magnesiumsulfat und Filtration wird im Vakuum eingeengt. Der so erhaltene Rückstand wird an Kieselgel mit Hexan/Methyl-tert.-butylether (97+3) chromatographiert. Man erhält so 181 g der Titelverbindung als farbloses Öl.
b) 2-(5-Diphenyl-tert.-butylsilyloxypentyl)-benzylalkohol
   Zu einer Lösung von 16,3 g 2-Methylbenzylalkohol tropft man unter Argon bei 0 °C 200 ml einer 1,6 molaren Butyllithiumlösung in Hexan. Nach beendeter Zugabe wird 5 Stunden unter Rückfluß erhitzt. Anschließend wird auf 25 °C abgekühlt und 51,0 g 4-Chlorbutan-1-ol-diphenyl-tert.-butylsilylether zugetropft. Nach 16 Stunden Rühren bei 25 °C verdünnt man mit Ether und wäscht einmal mit Wasser. Man trocknet über Magnesiumsulfat und dampft im Vakuum ein. Der so erhaltene Rückstand wird an Kieselgel chromatographiert. Mit Hexan/5-20 % Essigester erhält man 31,5 g der Titelverbindung als farbloses Öl.
   IR (CHCl₃): 3610, 3450 (breit), 2935 cm⁻¹.
c) 2-(5-Diphenyl-tert.-butylsilyloxypentyl)-benzaldehyd
   Zu einer Lösung von 21,4 g 2-(5-Diphenyl-tert.-butylsilyloxypentyl)-benzylalkohol in 600 ml Methylenchlorid gibt man 60,0 g Braunstein und rührt 4 Stunden bei 25 °C. Anschließend wird die Reaktionsmischung über Celite filtriert, gut mit Essigester nachgewaschen und im Vakuum eingeengt. Man erhält so 19,9 g der Titelverbindung als farbloses Öl.
   IR (CHCl₃): 2938, 2722, 1695, 1600 cm⁻¹.
d) (2E,4E)-5-[2-(5-Diphenyl-tert.-butylsilyloxypentyl)-phenyl]-2,4-pentadiensäureethylester
   Zu einer Lösung von 14 g Phosphonocrotonsäuretriethylester in 130 ml Tetrahydrofuran gibt man bei -20 °C unter Argon portionsweise insgesamt 5,9 g Kalium-tert.-butylat und läßt 30 Minuten bei -20 °C rühren. Dann tropft man 14,3 g 2-(5-Diphenyl-tert.-butylsilyloxypentyl)-benzaldehyd in 65 ml Tetrahydrofuran zu und rührt 45 Minuten bei -20 °C. Anschließend gibt man die Reaktionsmischung auf Wasser und extrahiert dreimal mit Methylenchlorid. Die organische Phase wird einmal mit Wasser gewaschen, über Natriumsulfat getrocknet und im Vakuum eingeengt. Der so erhaltene Rückstand wird mit Hexan/0-10 %. Methyl-tert.-butylether an Kieselgel chromatographiert. Man erhält 10,3 g der Titelverbindung als farbloses Öl.
   IR (CHCl₃): 2938, 1702, 1625, 998 cm⁻¹.
e) (2E,4E)-5-[2-(5-Diphenyl-tert.-butylsilyloxypentyl)-phenyl]-2,4-pentadien-1-ol
   Zu einer Lösung von 4,0 g (2E,4E)-5-[2-(5-Diphenyl-tert.-butylsilyloxypentyl)-phenyl]-2,4-pentadiensäureethylester in 100 ml Toluol tropft man bei -70 °C unter Argon 22 ml einer 1,2 molaren DIBAH-Lösung in Toluol zu. Nach 1 Stunde rühren bei -70 °C tropft man vorsichtig 3 ml Isopropanol gefolgt von 11 ml Wasser zur Reaktionsmischung und läßt 1 Stunde bei 24 °C rühren. Man filtriert, wäscht den Niederschlag gut mit Essigester und engt im Vakuum ein. Man erhält so 3,6 g der Titelverbindung als farbloses Öl.
   IR (CHCl₃): 3605, 3440 (breit), 2937, 990 cm⁻¹.
f) (2E,4E)-5-[2-(5-Diphenyl-tert.-butylsilyloxypentyl)-phenyl]-2,4-pentadienal
   Zu einer Lösung von (2E,4E)-5-[2-(5-Diphenyl-tert.-butylsilyloxypentyl)-phenyl]-2,4-pentadien-1-ol in 100 ml Methylenchlorid gibt man 25 g Braunstein und rührt 2 Stunden unter Argon bei 25 °C. Anschließend filtriert man über Celite ab, wäscht gut mit Methylenchlorid nach und engt im Vakuum ein. Man erhält so 3,5 g der Titelverbindung als farbloses Öl.
   IR (CHCl₃): 2938, 2745, 1680, 1620, 1602, 988 cm⁻¹.

### Beispiel 2

5-[2-[(1E,3E),-(5RS)-5-Acetoxy-1,3-undecadienyl]-phenyl]-pentan-1-ol

In Analogie zu Beispiel 1 erhält man aus 486 mg (2E,4E)-5-[2-(5-Diphenyl-tert.-butylsilyloxypentyl)-phenyl]-2,4-pentadienal (hergestellt in Beispiel 1) durch Umsetzung mit Hexylmagnesiumbromid-Lösung 398 mg 5-[2-[(1E,3E),-(5RS)-5-Acetoxy-1,3-undecadienyl]-phenyl]-pentan-1-ol-diphenyl-tert.-butylsilylether als farbloses Öl.

IR (CHCl₃): 3595, 3440 (breit), 2910, 980 cm⁻¹.

Durch Acetylierung erhält man aus 396 mg des vorstehend beschriebenen Alkohols 407 mg 5-[2-[(1E,3E)-(5RS)-5-Acetoxy-1,3-undecadienyl]-phenyl]-pentan-1-ol-diphenyl-tert.-butylsilylether als farbloses Öl.

IR (CHCl₃): 2938, 1730, 1245, 990 cm⁻¹.

Durch Silyletherspaltung erhält man ausgehend von 407 mg des zuvor hergestellten Acetats mit 1,97 g Tetrabutylammoniumfluoridtrihydrat 202 mg der Titelverbindung als farbloses Öl.

IR (CHCl₃): 3620, 3450 (breit), 2940, 1728, 1245, 990 cm⁻¹.

### Beispiel 3

5-[2-[(1E,3E)-(5RS)-5-Acetoxy-1,3-pentadecadienyl]-phenyl]-pentan-1-ol

In Analogie zu Beispiel 1 erhält man aus 497 mg (2E,4E)-5-[2-(5-Diphenyl-tert.-butylsilyloxypentyl)-phenyl]-2,4-pentadienal (hergestellt in Beispiel 1) durch Umsetzung mit Decylmagnesiumbromid-Lösung 592 mg 5-[2-[(1E,3E)-(5RS)-5-Hydroxy-1,3-pentadecadienyl]-phenyl]-pentan-1-ol-diphenyl-tert.-butylsilylether als farbloses Öl.

IR (CHCl₃): 3630, 3500 (breit), 2930, 990 cm⁻¹.

Durch Acetylierung erhält man aus 592 mg des vorstehend beschriebenen Alkohols 592 mg 5-[2-[(1E,3E)-(5RS)-5-Acetoxy-1,3-pentadecadienyl]-phenyl]-pentan-1-ol-diphenyl-tert.-butylsilylether als farbloses Öl.

IR (CHCl₃): 2930, 1730, 1248, 990 cm⁻¹.

Durch Silyletherspaltung erhält man ausgehend von 580 mg des zuvor hergestellten Acetats mit 2,85 g Tetrabutylammoniumfluoridtrihydrat 153 mg der Titelverbindung als farbloses Öl.

IR (CHCl₃): 3620, 3460 (breit), 2930, 1728, 1245, 990 cm⁻¹.

### Biespiel 4

5-[2-[(3E)-(5RS)-5-Acetoxy-3-tridecenyl]-phenyl]-pentan-1-ol

In Analogie zu Beispiel 1 erhält man aus 750 mg (4E)-5-[2-(5-Diphenyl-tert.-butylsilyloxypentyl)-phenyl]-2-pentenal durch Umsetzung mit Octylmagnesiumbromid-Lösung 845 mg 5-[2-[(3E)-(5RS)-5-Hydroxy-3-tridecenyl]-phenyl]-pentan-1-ol-diphenyl-tert.-butylsilylether als farbloses Öl.

IR (CHCl₃): 3622, 3480 (breit), 2930 cm⁻¹.

Durch Acetylierung erhält man aus 842 mg des vorstehend beschriebenen Alkohols 867 mg 5-[2-[(3E)-(5RS)-5-Acetoxy-3-tridecenyl]-phenyl]-pentan-1-ol-diphenyltert.-butylsilylether als farbloses Öl.

IR (CHCl₃): 2930, 1727, 1243 cm⁻¹.

Durch Silyletherspaltung erhält man ausgehend von 867mg des zuvor hergestellten Acetats mit 4,26 g Tetrabutylammoniumfluoridtrihydrat 287 mg der Titelverbindung als farbloses Öl.

IR (CHCl₃): 3620, 3460 (breit), 2938, 1725, 1250 cm⁻¹.

Das Ausgangsmaterial für die Titelverbindung in Beispiel 4 wird wie folgt hergestellt:
a) (2E)-3-[2-(5-Diphenyl-tert.-butylsilyloxypentyl)-phenyl]-2-propensäureethylester
   Zu einer Lösung von 4,78 g Phosphonoessigsäuretriethylester in 100 ml Tetrahydrofuran gibt man bei 0 °C unter Stickstoff portionsweise 1,96 g Kalium-tert.-butylat. Man rührt 10 Minuten bei 24 °C und tropft dann 5,0 g 2-(5-Diphenyl-tert.-butylsilyloxypentyl)-benzyldehyd (hergestellt in Beispiel 1) in 70 ml Toluol zu. Nach 5 Stunden Rühren bei 24 °C wird mit 800 ml Ether verdünnt und die organische Phase mit Wasser neutral gewaschen. Nach dem Trocknen über Natriumsulfat wird im Vakuum eingeengt. Das so erhaltene Rohprodukt wird an Kieselgel chromatographiert. Mit Hexan/0-20 % Essigester erhält man 5,8 g der Titelverbindung als farbloses Öl.
   IR (CHCl₃): 2938, 1710, 1633, 1603, 980 cm⁻¹.
b) (2E)-3-[2-(5-Diphenyl-tert.-butylsilyloxypentyl)-phenyl]-2-propen-1-ol
   Zu einer Lösung aus 5,78 g (2E)-3-[2-(5-Diphenyl-tert.-butylsilyloxypentyl)-phenyl]-2-propensäureethylester in 170 ml Toluol tropft man bei -70 °C unter Argon 33 ml einer 1,2 molaren DIBAH-Lösung in Toluol zu und rührt 1 Stunde bei -70 °C. Anschließend tropft man vorsichtig 10 ml Isopropanol gefolgt von 15 ml Wasser zu und rührt 1 Stunde bei 24 °C. Nach Filtration wäscht man den Niederschlag gut mit Essigester und engt im Vakuum ein. Das so erhaltene Rohprodukt chromatographiert man an Kieselgel. Mit Hexan/0-25 %. Essigester erhält man 4,94 g der Titelverbindung als farbloses Öl.
   IR (CHCl₃): 3608, 3440 (breit), 2936, 970 cm⁻¹.
c) 3-[2-(5-Diphenyl-tert.-butylsilyloxypentyl)-phenyl]-propan-1-ol
   Eine Lösung von 4,86 g (2E)-3-[2-(5-Diphenyl-tert.-butylsilyloxypentyl)-phenyl]-2-propen-1-ol in 120 ml Essigester wird mit 490 mg 10 % Palladium-Kohle versetzt und in einer Wasserstoffatmosphäre 5 Stunden bei 25 °C gerührt. Nach dem Abfiltrieren vom Katalysator engt man im Vakuum ein. Das so erhaltene Rohprodukt chromatographiert man an Kieselgel. Mit Hexan/0-20 % Essigester erhält man 4,6 g der Titelverbindung als farbloses Öl.
   IR (CHCl₃): 3620, 3450 (breit), 2938 cm⁻¹.
d) 3-[2-(5-Diphenyl-tert.-butylsilyloxypentyl)-phenyl]-propanal
   Zu einer Lösung vom 3,5 g 3-[2-(5-Diphenyl-tert.-butylsilyloxypentyl)-phenyl]-propan-1-ol in 200 ml Methylenchlorid gibt man bei 0 °C unter Argon portionsweise 35 g Collins-Reagenz und rührt 30 Minuten bei 24 °C. Anschließend gibt man in das Reaktionsgemisch Celite. Man filtriert über Celite ab, wäscht gut mit Methylenchlorid nach und engt im Vakuum ein. Das so erhaltene Rohprodukt wird mit Hexan/Essigester (9+1) an Kieselgel chromatographiert. Man erhält 2,5 g der Titelverbindung als farbloses Öl.
   IR (CHCl₃): 2938, 2742, 1728 cm⁻¹.
e) (2E)-5-[2-(5-Diphenyl-tert.-butylsilyloxypentyl)-phenyl]-2-pentensäureethylester
   Zu einer Lösung von 2,7 g Phosphonoessigsäuretriethylester in 50 ml Tetrahydrofuran gibt man bie 0 °C unter Stickstoff portionsweise 1,1 g Kalium-tert.-butylat. Man rührt 10 Minuten bei 24 °C und tropft dann 2,5 g 3-[2-(5-Diphenyltert.-butylsilyloxypentyl)-phenyl]-propanal in 40 ml Toluol zu. Nach 4 Stunden Rühren bei 24 °C wird mit 400 ml Ether verdünnt und die organische Phase mit Wasser neutral gewaschen. Nach dem Trocknen über Natriumsulfat wird im Vakuum eingeengt. Das so erhaltene Rohprodukt wird an Kieselgel chromatographiert. Mit Hexan/Essigester (95+5) erhält man 1,5 g der Titelverbindung als farbloses Öl.
   IR (CHCl₃): 2939, 1714, 1655, 975 cm⁻¹.
f) (2E)-5-[2-(5-Diphenyl-tert.-butylsilyloxypentyl)-phenyl]-2-penten-1-ol
   Zu einer Lösung von 1,0 g (2E)-5-[2-(5-Diphenyl-tert.-butylsilyloxypentyl)-phenyl]-2-pentensäureethylester in 33 ml Toluol tropft man bei -70 °C unter Argon 5,5 ml einer 1,2 molaren DIBAH-Lösung in Toluol zu und rührt 1 Stunde bei -70 °C. Anschließend tropft man vorsichtig 1 ml Isopropanol gefolgt von 3 ml Wasser zu und rührt 1 Stunde bei 25 °C. Nach Filtration wäscht man den Niederschlag gut mit Essigester und engt im Vakuum ein. Das so erhaltene Rohprodukt chromatographiert man an Kieselgel. Mit Hexan/0-25 % Essigester erhält man 870 mg der Titelverbindung als farbloses Öl.
   IR (CHCl₃): 3608, 3430 (breit), 2938, 972 cm⁻¹.
g) (2E)-5-[2-(5-Diphenyl-tert.-butylsilyloxypentyl)-phenyl]-2-pentenal
   Zu einer Lösung von 870 mg (2E)-5-[2-(5-Diphenyl-tert.-butylsilyloxypentyl)-phenyl]-2-penten-1-ol in 30 ml Methylenchlorid gibt man 6,0 g Braunstein und rührt 2 Stunden unter Argon bei 25 °C. Anschließend filtriert man über Celite ab, wäscht gut mit Methylenchlorid nach und engt im Vakuum ein. Das so erhaltene Rohprodukt wird an Kieselgel mit Hexan/0-10 % Essigester chromatographiert. Man erhält 750 mg der Titelverbindung als farbloses Öl.
   IR (CHCl₃): 2937, 2742, 1690, 1638, 875 cm⁻¹.

### Beispiel 5

5-[2-[(1E,3E)-(5RS)-5-Acetoxy-1,3-tridecadienyl]-phenyl]-pentansäure

Zu einer Lösung von 92 mg 5-[2-[(1E,3E)-(5RS)-5-Acetoxy-1,3-tridecadienyl]-phenyl]-pentan-1-ol (hergestellt in Beispiel 1) in 10 ml Methylenchlorid gibt man 750 mg Collins-Reagenz und läßt 40 Minuten bei 0 °C unter Argon rühren. Anschließend versetzt man Celite und filtriert über Celite ab, wobei mit einer Mischung aus Hexan**/**EE (1+1) gut nachgewaschen wird. Nach dem Einengen im Vakuum erhält man 92 mg 5-[2-[(1E,3E)-(5RS)-5-Acetoxy-1,3-tridecadienyl]-phenyl]-pentanal, welcher ohne weitere Reinigung in die nächste Stufe eingesetzt wird.

IR (CHCl₃): 2930, 2735, 1727, 1245, 990 cm⁻¹.

Zu einer Lösung von 92 mg des vorstehend hergestellten Aldehyds in 8 ml Aceton tropft man unter Rühren bei -30 °C 0,2 ml Jones-Reagenz (J. Chem. Soc. 1953, 2555) und rührt 15 Minuten bei -30 °C. Anschließend gibt man 0,1 ml Isopropanol hinzu und rührt 5 Minuten bei -20 °C. Dann verdünnt man mit Ether, wäscht mit ges. Natriumchlorid-Lösung neutral und trocknet über Natriumsulfat. Nach dem Einengen im Vakuum wird der Rückstand an Kieselgel chromatographiert. Mit Hexan**/**30-50 **%** Essigester erhält man 53,8 mg der Titelverbindung als farbloses Öl.

IR (CHCl₃): 3520, 3350 (breit), 2930, 1724, 1245, 988 cm⁻¹.

### Biespiel 6

5-[2-[(1E,3E)-(5RS)-5-Acetoxy-1,3-undecadienyl]-phenyl]-pentansäure

In Analogie zu Beispiel 5 erhält man aus 180 mg 5-[2-[(1E,3E)-(5RS)-5-Acetoxy-1,3-tridecadienyl]-phenyl]-pentan-1-ol (hergestellt in Beispiel 2) 179 mg 5-[2-[(1E,3E)-(5RS)-5-Acetoxy-1,3-tridecadienyl]-phenyl]-pentanal.

IR (CHCl₃): 2938, 2730, 1725, 1245, 990 cm⁻¹.

Ausgehend von den 179 mg des vorstehend hergestellten Aldehyds erhält man in Analogie zu Beispiel 5 71 mg der Titelverbindunge als farbloses Öl.

IR (CHCl₃): 3520, 3350 (breit), 2938, 1728, 1245, 988 cm⁻¹.

### Biespiel 7

5-[2-[(1E,3E)-(5RS)-5-Acetoxy-1,3-pentadecadienyl]-phenyl]-pentansäure

In Analogie zu Beispiel 5 erhält man aus 140 mg 5-[2-[(1E,3E)-(5RS)-5-Acetoxy-1,3-pentadecadienyl]-phenyl]-pentan-1-ol (hergestellt in Beispiel 3) 139 mg 5-[2-[(1E,3E)-(5RS)-5-Acetoxy-1,3-pentadecadienyl]-phenyl]-pentanal

IR(CHCl₃): 2938, 2732, 1725, 1245, 990 cm⁻¹

Ausgehend von den 139 mg des vorstehend hergestellten Aldehyds erhält man in Analogie zu Beispiel 5 75 mg der Titelverbindung als fabloses Öl.

IR (CHCl₃): 3520, 3350 (breit), 2930, 1723, 1745, 988 cm⁻¹.

### Beispiel 8

5-[2-[(3E)-(5RS)-5-Acetoxy-3-tridecenyl]-phenyl]-pentansäure

In Analogie zu Beispiel 5 erhält man aus 150 mg 5-[2-[(3E)-(5RS)-5-Acetoxy-3-tridecenyl]-phenyl]-pentan-1-ol (hergestellt in Beispiel 4) 148 mg 5-[2-[(3E)-(5RS)-5-Acetoxy-3-tridecenyl]-phenyl]-pentanal.

IR (CHCl₃): 2930, 2728, 1723, 1250, 970 cm⁻¹.

Ausgehend von den 148 mg des vorstehend hergestellten Aldehyds erhält man in Analogie zu Beispiel 5 55 mg der Titelverbindung als farbloses Öl.

IR (CHCl₃): 3520, 3350 (breit), 2932, 136, 1709, 1240, 970 cm⁻¹.

### Biespiel 9

5-[2-[(1E,3E)-(5R5)-5-Hydroxy-1,3-tridecadienyl]-phenyl]-pentansäure

Zu einer Lösung von 12 mg 5-[2-[(1E,3E)-(5RS)-5-Acetoxy-1,3-tridecadienyl]-phenyl]-pentansäure (hergestellt in Beispiel 5) in 0,3 ml Methanol gibt man bei 25 °C 0,3 ml einer 0,5 normalen Natronlauge und rührt 4 Stunden bei 24 °C. Anschließend kühlt man auf 0 °C und säuert mit einer 0,5 normalen Schwefelsäure auf pH=6 an. Man extrahiert viermal mit Essigester, wäscht die organische Phase mit Wasser und trocknet über Natriumsulfat. Man engt im Vakuum ein und chromatographiert den Rückstand an Kieselgel. Mit Ether/Hexan (7+3) als Laufmittel erhält man 3,8 mg der Titelverbindung als farbloses Öl.

IR (CHCl₃): 3600, 3520, 3400 (breit), 2928, 1710, 988 cm⁻¹.

### Biespiel 10

5-[2-[(1E,3E)-(5RS)-5-Hydroxy-1,3-undecadienyl]-phenyl]-pentansäure

In Analogie zu Beispiel 9 erhält man aus 60 mg 5-[2-[(1E,3E)-(5RS)-5-Acetoxy-1,3-undecadienyl]-phenyl]-pentansäure (hergestellt in Beispiel 6) 28 mg der Titelverbindung als farbloses Öl.

IR (KBr: 3410 (breit), 2930, 1708, 988 cm⁻¹.

### Biespiel 11

5-[2-[(1E.3E)-(5RS)-5-Hydroxy-1,3-pentadecadienyl]-phenyl]-pentansäure

In Analogie zu Beispiel 9 erhält man aus 62 mg 5-[2-[(1E,3E)-(5RS)-5-Acetoxy-1,3-pentadecadienyl]-phenyl]-pentansäure (hergestellt in Beispiel 7) 15 mg der Titelverbindung als farbloses Öl.

IR (CHCl₃): 3600, 3520, 3400 (breit), 293, 171, 990 cm⁻¹.

### Biespiel 12

5-[2-[(3E)-(5RS)-5-Hydroxy-3-tridecenyl]-phenyl]-pentansäure

In Analogie zu Beispiel 9 erhält man aus 45 mg 5-[2-[(3E)-(5RS)-5-Acetoxy-3-tridecenyl]-phenyl]-pentansäure (hergestellt in Beispiel 8) 32 mg der Titelverbindung als farbloses Öl.

IR (CHCl₃): 3605, 3520, 3400 (breit), 2932, 1712, 972 cm⁻¹.

### Biespiel 13

5-[2-[(1E,3E)-(5RS)-5-Hydroxy-1,3-tridecadienyl]-phenyl]-pentan-1-ol

Zu einer Lösung von 270 mg 5-[2-[(1E,3E)-(5RS)-5-Acetoxy-1,3-tridecadienyl]-phenyl]-pentan-1-ol (hergestellt in Beispiel 1) in 11,7 ml Methanol gibt man 6,7 ml einer 0,5 normalen Natronlauge und rührt die Reaktionsmischung 16 Stunden bei 25 °C. Anschließend kühlt man auf 0 °C und säuert mit einer 0,5 normalen Schwefelsäure auf pH=6 an. Man extrahiert dreimal mit Methylenchlorid und trocknet die organische Phase über Natriumsulfat. Man engt im Vakuum ein und chromatographiert den Rückstand an Kieselgel. Mit Hexan/50-80 % Essigester als Laufmittel erhält man 184 mg der Titelverbindung als farbloses Öl.

IR (CHCl₃): 3350 (breit), 2930, 988 cm⁻¹.

### Biespiel 14

5-[2-[(1E,3E)-(5RS)-5-Hydroxy-1,3-tridecadienyl]-phenyl]-pentansäuremethylester

Zu einer Lösung von 5 mg 5-[2-[(1E,3E)-(5RS)-5-Hydroxy-1,3-tridecadienyl]-phenyl]-pentansäure (hergestellt in Beispiel 9) in 0,5 ml Methylenchlorid gibt man bei 0 °C eine etherische Diazomethanlösung bis zur bleibenden Gelbfärbung und rührt 5 Minuten bei 0 °C. Anschließend engt man im Vakuum ein und chromatographiert den Rückstand an Kieselgel. Mit Hexan/0-80 % Ether erhält man 2,6 mg der Titelverbindung als farbloses Öl.

IR (CHCl₃): 3620, 3480 (breit), 2930, 1730, 988 cm⁻¹.

### Beispiel 15

5-[2-[(1E,3E)-(5RS)-5-Hydroxy-1,3-tridecadienyl]-phenyl]-pentansäureethylester

In Analogie zu Beispiel 14 erhält man aus 3,7 mg der in Beispiel 9 hergestellten Säure mit Diazoethan 1,8 mg der Titelverbindung als farbloses Öl.

IR (CHCl₃): 3620, 3480 (breit), 2930, 1732, 990 cm⁻¹.

### Biespiel 16

5-[2-[(1E,3E)-(5RS)-5-Hydroxy-1,3-tridecadienyl]-phenyl]-pentansäure-tris-(hydroxymethyl)-aminomethan-salz

Zu einer Lösung von 13 mg der nach Beispiel 9 hergestellten Säure in 2 ml Acetonitril gibt man bei 70 °C eine Lösung von 5 mg Tris-(hydroxymethyl)-aminomethan in 0,02 ml Wasser. Man läßt unter Rühren abkühlen, dekantiert nach 16 Stunden vom Lösungsmittel und trocknet den Rückstand im Vakuum. Man isoliert 9 mg der Titelverbindung als wachsartige Masse.

### Beispiel 17

5-[2-[(5RS)-5-Hydroxy-tridecanyl]-phenyl]-pentansäure

Eine Lösung von 60 mg der nach Beispiel 9 hergestellten Säure in 4 ml Essigester versetzt man mit 12 mg Palladium 10 %ig auf Kohle und rührt die Suspension 3 Stunden bei 24 °C in einer Wasserstoffatmosphäre. Anschließend filtriert man vom Katalysator ab und engt im Vakuum ein. Den so erhaltenen Rückstand chromatographiert man an Kieselgel. Mit Ether/Hexan (7+3) als Laufmittel erhält man 38 mg der Titelverbindung als farbloses Öl.

IR (CHCl₃): 3600, 3440 (breit), 2925, 1710 cm⁻¹.

### Beispiel 18

5-[2-[(5RS)-5-Acetoxy-tridecanyl]-phenyl]-pentansäure

In Analogie zu Beispiel 17 erhält man aus 46 mg der in Beispiel 5 hergestellten Säure 29 mg der Titelverbindung als farbloses Öl.

IR (CHCl₃): 3600, 3520, 3350, 2930, 1722, 1712, 1255 cm⁻¹.

## Patentansprüche

1. Leukotrien-B₄-Analoga der Formel I, 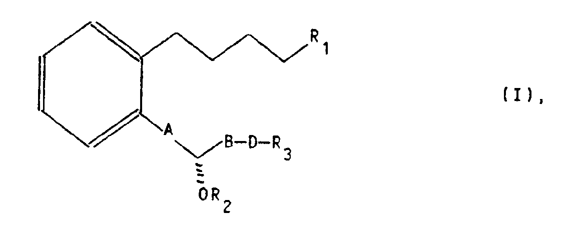 worin
R₁ die Reste CH₂OH, CH₃, CF₃, oder COOR₄ mit R₄ in der Bedeutung eines Wasserstoffatoms, eines Alkylrestes mit 1-10 C-Atomen, eines Cycloalkylrestes mit 3-10 C-Atomen, eines gegebenenfalls durch 1-2 Chlor, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrestes mit 6-10 C-Atomen, eines -CH₂-CO-Aryl-Restes mit 6-10 C-Atomen für Aryl oder eines 5-6-gliedrigen heterocyclischen Restes mit wenigstens 1 Heteroatom, den Rest CONHR₅ mit R₅ in der Bedeutung des Restes R₄ oder eines Alkanoyloder Alkansulfonylrestes mit 1-10 C-Atomen,
A eine trans,trans-CH=CH-CH=CH-, trans-CH₂-CH₂-CH=CH- oder Tetramethylengruppe,
B eine geradkettige oder verzweigtkettige gesättigte oder ungesättigte Alkylengruppe mit bis zu 10 C-Atomen, die gegebenenfalls durch Fluor substituiert sein kann, oder die Gruppe 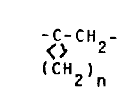 mit n = 1, 2 oder 3,
D eine Direktverbindung, Sauerstoff, Schwefel, eine -C≡C-Gruppe oder eine -CH=CR₆-Gruppe mit R₆ als Wasserstoff, C₁-C₅-Alkyl, Chlor oder Brom,
B und D gemeinsam eine direkte Bindung,
R₂ ein Wasserstoffatom oder einen Säurerest einer organischen Säure mit 1-15 C-Atomen und
R₃ ein Wasserstoffatom, einen Alkylrest mit 1-10 C-Atomen, einen durch Chlor oder Brom substituierten Alkylrest mit 1-10 C-Atomen, einen Cycloalkylrest mit 3-10 C-Atomen, einen gegebenenfalls durch 1-2 Chlor-, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrest mit 6-10 C-Atomen oder einen 5-6-gliedrigen heterocyclischen Rest mit wenigstens 1 Heteroatom bedeuten und, falls R₄ die Bedeutung eines Wasserstoffatom hat, deren Salze mit physiologisch verträglichen Basen und deren Cyclodextrinclathrate.

2. Verfahren zur Herstellung der Leukotrien-B₄-Derivate der Formel I, dadurch gekennzeichnet, daß man einen Aldehyd der Formel II, 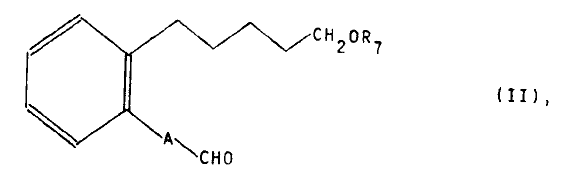
worin A die oben angegebene Bedeutung hat und R₇ einen leicht abspaltbaren Etherrest aus der Gruppe Dimethyl-tert.-butylsilyl, Trimethylsilyl-, Tribenzylsilyl-, Diphenyl-tert.-butylsilyl-, Tetrahydropyranyl-, Tetrahydrofuranyl- und α-Ethoxyethyl bedeutet, mit einer magnesiumorganischen Verbindung der Formel III,
X - Mg - B - D - R₃ (III),
worin X Chlor, Brom oder Jod und B, D und R₃ die oben angegebene Bedeutungen haben, umgesetzt und gegebenenfalls anschließend in beliebiger Reihenfolge Enantiomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R₁=COOR₅) verseift und/oder reduziert und/oder eine Carboxylgruppe (R₅=H) verestert und/oder eine freie Carboxylgruppe (R₅=H) in ein Amid (R₁=CONHR₆) überführt oder eine Carboxygruppe mit einer physiologisch verträglichen Base in ein Salz überführt.

3. 5-[2-[(1E,3E)-(5RS)-5-Acetoxy-1,3-tridecadienyl]-phenyl]-pentan-1-ol

4. 5-[2-[(1E,3E),-(5RS)-5-Acetoxy-1,3-undecadienyl]-phenyl]-pentan-1-ol

5. 5-[2-[(1E,3E)-(5RS)-5-Acetoxy-1,3-pentadecadienyl]-phenyl]-pentan-1-ol

6. 5-[2-[(3E)-(5RS)-5-Acetoxy-3-tridecenyl]-phenyl]-pentan-1-ol

7. 5-[2-[(1E,3E)-(5RS)-5-Acetoxy-1,3-tridecadienyl]-phenyl]-pentansäure

8. 5-[2-[(1E,3E)-(5RS)-5-Acetoxy-1,3-undecadienyl]-phenyl]-pentansäure

9. 5-[2-[(1E,3E)-(5RS)-5-Acetoxy-1,3-pentadecadienyl]-phenyl]-pentansäure

10. 5-[2-[(3E)-(5RS)-5-Acetoxy-3-tridecenyl]-phenyl]-pentansäure

11. 5-[2-[(1E,3E)-(5RS)-5-Hydroxy-1,3-tridecadienyl]-phenyl]-pentansäure

12. 5-[2-[(1E,3E)-(5RS)-5-Hydroxy-1,3-undecadienyl]-phenyl]-pentansäure

13. 5-[2-[(1E,3E)-(5RS)-5-Hydroxy-1,3-pentadecadienyl]-phenyl]-pentansäure

14. 5-[2-[(3E)-(5RS)-5-Hydroxy-3-tridecenyl]-phenyl]-pentansäure

15. 5-[2-[(1E,3E)-(5RS)-5-Hydroxy-1,3-tridecadienyl]-phenyl]-pentan-1-ol

16. 5-[2-[(1E,3E)-(5RS)-5-Hydroxy-1,3-tridecadienyl]-phenyl]-pentansäuremethylester

17. 5-[2-[(1E,3E)-(5RS)-5-Hydroxy-1,3-tridecadienyl]-phenyl]-pentansäureethylester

18. 5-[2-[(1E,3E)-(5RS)-5-Hydroxy-1,3-tridecadienyl]-phenyl]-pentansäure-tris-(hydroxymethyl)-aminomethan-salz

19. 5-[2-[(5RS)-5-Hydroxy-tridecanyl]-phenyl]-pentansäure

20. 5-[2-[(5RS)-5-Acetoxy-tridecanyl]-phenyl]-pentansäure

21. Arzneimittel bestehend aus einer oder mehreren Verbindungen gemäß Anspruch 1 und üblichen Hilfs- und Trägerstoffen.

## Claims

1. Leucotriene B₄ analogues of the formula I 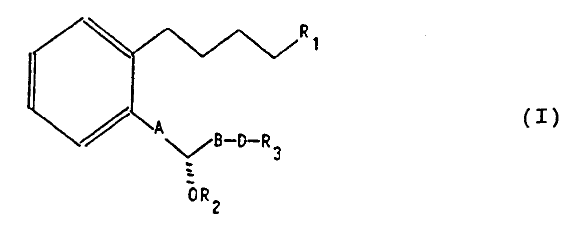 wherein
R₁ represents the radicals CH₂OH, CH₃, CF₃, or COOR₄ in which R₄ represents a hydrogen atom, an alkyl radical having 1-10 carbon atoms, a cycloalkyl radical having 3-10 carbon atoms, an aryl radical having 6-10 carbon atoms that is optionally mono- or di-substituted by chlorine, bromine, phenyl, alkyl having 1-4 carbon atoms, chloromethyl, fluoromethyl, trifluoromethyl, carboxy, C₁-C₄-alkoxy or by hydroxy, a CH₂-CO-aryl radical having 6-10 carbon atoms for aryl or a 5- or 6-membered heterocyclic radical having at least 1 hetero atom, or R₁ represents the radical CONHR₅ in which R₅ represents a radical R₄ or an alkanoyl or alkanesulphonyl radical having 1-10 carbon atoms,
A represents a trans,trans-CH=CH-CH=CH-, trans-CH₂-CH₂-CH=CH- or tetramethylene group,
B represents a straight-chain or branched saturated or unsaturated alkylene group having up to 10 carbon atoms which may optionally be substituted by fluorine, or represents the group 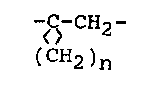 in which n = 1, 2 or 3,
D represents a direct bond, oxygen, sulphur, a -C≡C- group or a -CH=CR₆ group in which R₆ represents hydrogen, C₁-C₅-alkyl, chlorine or bromine,
B and D together represent a direct bond,
R₂ represents a hydrogen atom or an acid residue of an organic acid having 1-15 carbon atoms, and
R₃ represents a hydrogen atom, an alkyl radical having 1-10 carbon atoms, an alkyl radical having 1-10 carbon atoms that is substituted by chlorine or bromine, a cycloalkyl radical having 3-10 carbon atoms, an aryl radical having 6-10 carbon atoms that is optionally mono- or di-substituted by chlorine, bromine, phenyl, alkyl having 1-4 carbon atoms, chloromethyl, fluoromethyl, trifluoromethyl, carboxy, C₁-C₄-alkoxy or by hydroxy, or a 5- or 6-membered heterocyclic radical having at least 1 hetero atom, and, if R₄ represents a hydrogen atom, their salts with physiologically tolerable bases and their cyclodextrin clathrates.

2. Process for the manufacture of the leucotriene B₄ derivatives of the formula I, characterised in that an aldehyde of the formula II 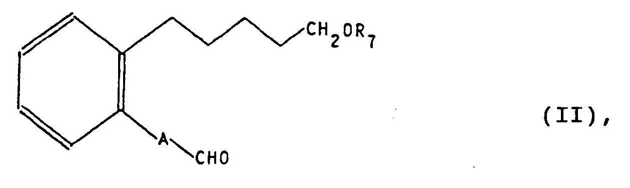 wherein A has the meanings given above and R₇ is a readily removable ether radical from the group dimethyl-tert.-butylsilyl, trimethylsilyl, tribenzylsilyl, diphenyl-tert.-butylsilyl, tetrahydropyranyl, tetrahydrofuranyl and α-ethoxyethyl, is reacted with an organomagnesium compound of the formula III
X - Mg - B - D - R₃ (III),
wherein X represents chlorine, bromine or iodine and B, D and R₃ have the meanings given above, and then optionally, in any desired sequence, enantiomers are separated, protected hydroxy groups are freed and/or a free hydroxy group is esterified and/or the 1-hydroxy group is oxidised to carboxylic acid and/or double bonds are hydrogenated and/or an esterified carboxy group (R₁ = COOR₅) is hydrolysed and/or reduced and/or a carboxy group (R₅ = H) is esterified and/or a free carboxy group (R₅ = H) is converted into an amide (R₁ = CONHR₆), or a carboxy group is converted into a salt with a physiologically tolerable base.

3. 5-[2-[(1E,3E)-(5RS)-5-acetoxy-1,3-tridecadienyl]-phenyl]-pentan-1-ol.

4. 5-[2-[(1E,3E)-(5RS)-5-acetoxy-1,3-undecadienyl]-phenyl]-pentan-1-ol.

5. 5-[2-[(1E,3E)-(5RS)-5-acetoxy-1,3-pentadecadienyl]-phenyl]-pentan-1-ol.

6. 5-[2-[(3E)-(5RS)-5-acetoxy-3-tridecenyl]-phenyl]-pentan-1-ol.

7. 5-[2-[(1E,3E)-(5RS)-5-acetoxy-1,3-tridecadienyl]-phenyl]-pentanoic acid.

8. 5-[2-[(1E,3E)-(5RS)-5-acetoxy-1,3-undecadienyl]-phenyl]-pentanoic acid.

9. 5-[2-[(1E,3E)-(5RS)-5-acetoxy-1,3-pentadecadienyl]-phenyl]-pentanoic acid.

10. 5-[2-[(3E)-(5RS)-5-acetoxy-3-tridecenyl]-phenyl]-pentanoic acid.

11. 5-[2-[(1E,3E)-(5RS)-5-hydroxy-1,3-tridecadienyl]-phenyl]-pentanoic acid.

12. 5-[2-[(1E,3E)-(5RS)-5-hydroxy-1,3-undecadienyl]-phenyl]-pentanoic acid.

13. 5-[2-[(1E,3E)-(5RS)-5-hydroxy-1,3-pentadecadienyl]-phenyl]-pentanoic acid.

14. 5-[2-[(3E)-(5RS)-5-hydroxy-3-tridecenyl]-phenyl]-pentanoic acid.

15. 5-[2-[(1E,3E)-(5RS)-5-hydroxy-1,3-tridecadienyl]-phenyl]-pentan-1-ol.

16. 5-[2-[(1E,3E)-(5RS)-5-hydroxy-1,3-tridecadienyl]-phenyl]-pentanoic acid methyl ester.

17. 5-[2-[(1E,3E)-(5RS)-5-hydroxy-1,3-tridecadienyl]-phenyl]-pentanoic acid ethyl ester.

18. 5-[2-[(1E,3E)-(5RS)-5-hydroxy-1,3-tridecadienyl]-phenyl]-pentanoic acid tris(hydroxymethyl)aminomethane salt.

19. 5-[2-[(5RS)-5-hydroxy-tridecanyl]-phenyl]-pentanoic acid.

20. 5-[2-[(5RS)-5-acetoxy-tridecanyl]-phenyl]-pentanoic acid.

21. Medicament consisting of one or more compounds according to claim 1 and customary adjuvants and carriers.

## Revendications

1. Analogues du leucotriène-B₄ répondant à la formule I : 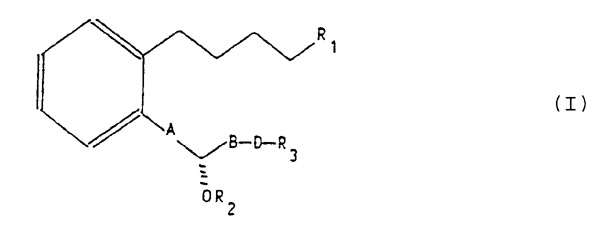 dans laquelle
R₁ désigne les radicaux CH₂OH, CH₃, CF₃ ou COOR₄, R₄ désignant un atome d'hydrogène, un radical alkyle en C₁-C₁₀, un radical cycloalkyle C₃-C₁₀, un radical aryle en C₆-C₁₀ éventuellement substitué par un ou deux radicaux chlore, brome, phényle, alkyle en C₁-C₄, chlorométhyle, fluorométhyle, trifluorométhyle, carboxy, alcoxy en C₁-C₄ ou hydroxy, un radical -CH₂-CO-aryle ayant 6 à 10 atomes de carbone dans le groupe aryle ou un radical hétérocyclique à 5 ou 6 maillons ayant au moins un hétéroatome, le radical CONHR₅ dans lequel R₅ désigne le radical R₄ ou un radical alcanoyle ou alcane-sulfonyle en C₁-C₁₀,
A désigne un groupe trans,trans-CH=CH-CH=CH-, trans-CH₂-CH₂-CH=CH- ou tétraméthylène,
B désigne un groupe alkylène linéaire ou ramifié, saturé ou insaturé ayant jusqu'à 10 atomes de C, qui peut le cas échéant être substitué par l'atome de fluor, ou par le groupe 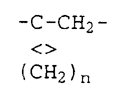 avec n = 1, 2 ou 3,
D est une liaison directe, un atome d'oxygène, de soufre, un groupe -C≡C- ou un groupe -CH=CR₆ - dans lequel R₆ désigne un atome d'hydrogène, un groupe alkyle en C₁-C₅, un atome de chlore ou de brome,
B et D désignent ensemble une liaison directe,
R₂ est un atome d'hydrogène ou un radical acide d'un acide organique en C₁-C₁₅, et
R₃ est un atome d'hydrogène, un radical alkyle en C₁-C₁₀, un radical alkyle en C₁-C₁₀ substitué par des atomes de chlore ou de brome, un radical cycloalkyle en C₃-C₁₀, un radical aryle en C₆-C₁₀ éventuellement substitué par un ou deux radicaux chlore, brome, phényle, alkyle en C₁-C₄, chlorométhyle, fluorométhyle, trifluorométhyle, carboxy, alcoxy en C₁-C₄ ou hydroxy ou un radical hétérocyclique à 5 ou 6 maillons avec au moins un hétéroatome, et, dans le cas où R₄ représente un atome d'hydrogène, leurs sels avec des bases physiologiquement acceptables et leurs clathrates avec la cyclodextrine.

2. Procédé de préparation des dérivés du leucotriène-B₄ répondant à la formule I, caractérisé en ce qu'on fait réagir un aldéhyde répondant à la formule II : 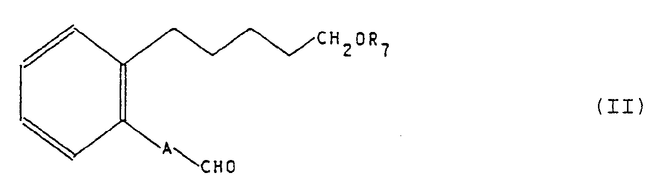 dans laquelle A a la signification indiquée ci-dessus et R₇ désigne un radical d'éther aisément éliminable choisi parmi les radicaux diméthyl-tert-butylsilyle, triméthylsilyle, tribenzylsilyle, diphényl-tert-butylsilyle, tétrahydropyranyle, tétrahydrofuranyle et α-éthoxyéthyle, avec un composé organique du magnésium répondant à la formule III :
X - Mg - B - D - R₃ (III)
dans laquelle X désigne un atome de chlore, de brome ou d'iode et B, D et R₃ ont les significations indiquées ci-dessus, puis le cas échéant, dans un ordre quelconque, on sépare les énantiomères, on libère les groupes hydroxy protégés et/ou on estérifie un groupe hydroxy libre et/ou on oxyde le groupe 1-hydroxy en acide carboxylique et/ou on hydrogène les doubles liaisons et/ou on saponifie et/ou on réduit un groupe carboxyle estérifié (R₁ = COOR₅ et/ou on estérifie un groupe carboxyle (R₅ = H) et/ou on transforme un groupe carboxyle libre (R₅ = H) en un amide (R₁ = CONHR₆), ou bien on transforme un groupe carboxy en un sel avec une base physiologiquement acceptable.

3. 5-[2-[(1E,3E)-(5RS)-5-Acétoxy-1,3-tridécadiényl]-phényl]-pentan-1-ol

4. 5-[2-[(1E,3E)-(5RS)-5-Acétoxy-1,3-undécadiényl]-phényl]-pentan-1-ol

5. 5-[2-[(1E,3E)-(5RS)-5-Acétoxy-1,3-pentadécadiényl]-phényl]-pentan-1-ol

6. 5-[2-[(3E)-(5RS)-5-Acétoxy-3-tridécényl]-phényl]-pentan-1-ol

7. Acide 5-[2-[(1E,3E)-(5RS)-5-acétoxy-1,3-tridécadiényl]-phényl]-valérique

8. Acide 5-[2-[(1E,3E)-(5RS)-5-acétoxy-1,3-undécadiényl]-phényl]-valérique

9. Acide 5-[2-[(1E,3E)-(5RS)-5-acétoxy-1,3-pentadécadiényl]-phényl]-valérique

10. Acide 5-[2-[(3E)-(5RS)-5-acétoxy-3-tridécényl]-phényl]-valérique

11. Acide 5-[2-[(1E,3E)-(5RS)-5-hydroxy-1,3-tridécadiényl]-phényl]-valérique

12. Acide 5-[2-[(1E,3E)-(5RS)-5-hydroxy-1,3-undécadiényl]-phényl]-valérique

13. Acide 5-[2-[(1E,3E)-(5RS)-5-hydroxy-1,3-pentadécadiényl]-phényl]-valérique

14. Acide 5-[2-[(3E)-(5RS)-5-hydroxy-3-tridécényl]-phényl]-valérique

15. 5-[2-[(1E,3E)-(5RS)-5-Hydroxy-1,3-tridécadiényl]-phényl]-pentan-1-ol

16. 5-[2-[(1E,3E)-(5RS)-5-Hydroxy-1,3-tridécadiényl]-phényl]-valérate de méthyle

17. 5-[2-[(1E,3E)-(5RS)-5-Hydroxy-1,3-tridécadiényl]-phényl]-valérate d'éthyle

18. 5-[2-[(1E,3E)-(5RS)-5-Hydroxy-1,3-tridécadiényl]-phényl]-valérate de tris-(hydroxyméthyl)-aminométhane

19. Acide 5-[2-[(5RS)-5-hydroxy-tridécanyl]-phényl]-valérique

20. Acide 5-[2-[(5RS)-5-acétoxy-tridécanyl]-phényl]-valérique

21. Médicament constitué d'un ou plusieurs composés selon la revendication 1, de substances auxiliaires et de supports ordinaires.
